# EUROPEAN PATENT SPECIFICATION

(11) **EP 4 385 504 B1**
(45) Date of publication and mention of the grant of the patent: **18.11.2026**
(21) Application number: 22855499.4
(22) Date of filing: 11.08.2022
(51) Int. Cl.: A61K 31/05, A61K 9/127, A61P 35/00

(54) **USE OF HONOKIOL IN PREPARATION OF DRUG FOR TREATING MENINGIOMA**
VERWENDUNG VON HONOKIOL ZUR HERSTELLUNG EINES ARZNEIMITTELS ZUR BEHANDLUNG VON MENINGIOMEN
UTILISATION D'HONOKIOL DANS LA PRÉPARATION D'UN MÉDICAMENT POUR LE TRAITEMENT D'UN MÉNINGIOME

(30) Priority: 12.08.2021 CN 202110924249
(43) Date of publication of application: 19.06.2024
(73) Proprietor: CHENGDU JINRUI FOUNDATION BIOTECH CO., LTD., Chengdu, Sichuan 610000 (CN)
(72) Inventor: LI, Wenbin, Chengdu, Sichuan 610000 (CN); WANG, Ce, Chengdu, Sichuan 610000 (CN); LAI, Xintian, Chengdu, Sichuan 610000 (CN); WANG, Xiaobo, Chengdu, Sichuan 610000 (CN)
(74) Representative: Dehns
(86) International application number: PCT/CN2022/111765
(87) International publication number: WO 2023/016519

(56) References cited:
- CN-A- 101 742 991
- CN-A- 102 178 666
- CN-A- 112 076 179
- US-A1- 2013 129 809
- MAROSI C ET AL: "Meningioma", CRITICAL REVIEWS IN ONCOLOGY/HEMATOLOGY, ELSEVIER, AMSTERDAM, NL, vol. 67, no. 2, 1 August 2008 (2008-08-01), pages 153 - 171, XP022778124, ISSN: 1040-8428, [retrieved on 20080314], DOI: 10.1016/J.CRITREVONC.2008.01.010
- ROTH P ET AL: "Chemotherapy of brain tumors in aduts", DER NERVENARZT, SPRINGER VERLAG, BERLIN, DE, vol. 86, no. 4, 19 March 2015 (2015-03-19), pages 495 - 508, XP035478916, ISSN: 0028-2804, [retrieved on 20150319], DOI: 10.1007/S00115-014-4242-0
- DONG YI: "The Effects and Mechanisms of Honokiol on Glioma U87Cell Growth", CHINA MASTER'S' THESES FULL-TEXT DATABASE (ELECTRONIC JOURNAL)-INFORMATION & TECHNOLOGY), TIANJIN POLYTECHNIC UNIVERSITY, CN, no. 3, 15 March 2013 (2013-03-15), CN , XP093034323, ISSN: 1674-0246
- YANG LIHUA, MA CHUN, LI SHULING, CHEN SHUANG: "The Mechanism of Neuroprotective Action of Phylosome with Lyophilized Liposomes Injected by Honokiol in Rats with Vascular Dementia", JOURNAL OF SHAANXI UNIVERSITY OF CHINESE MEDICINE, vol. 40, no. 4, 10 July 2017 (2017-07-10), pages 114 - 119, XP093034330, ISSN: 2096-1340, DOI: 10.13424/j.cnki.jsctcm.2017.04.036

## Description

### Technical Field

The present invention relates to a medical use of honokiol. Specifically, the present invention relates to honokiol for use in the treatment of meningioma.

### Background

The meningioma is the most common primary tumor of the central nervous system, mostly originating from the endothelial cells of the arachnoid, mainly occurring in the granules or villus of the arachnoid. The meningioma accounts for about 36.6% of all primary central nervous system tumors and 53.2% of non-malignant primary central nervous system tumors. Meningiomas are divided into benign meningiomas and malignant meningiomas. The typical clinical symptoms of the meningioma are increased intracranial pressure, focal nervous system (including cranial nerve) defects, and generalized and partial epileptic seizures caused by focal mass effect, the most common of which are headache, epileptic seizures, visual symptoms, limb weakness, and altered mental status.

The incidence of the meningioma has a tendency to increase year by year, from 4.52/100,000 during 1998~2002 to 8.3/100,000 during 2010~2014. The onset of the meningioma is more common in elderly patients, mostly 70 to 80 years old.

Ionizing radiation is a risk factor for the development of the meningioma. In Hiroshima and Nagasaki, 88 meningiomas were observed among 80,160 atomic bomb survivors. Even very small doses of radiation to the head (eg, those used in dental radiography) may increase the risk of the development of the meningioma. However, there is no clear dose-response relationship between ionizing radiation and the incidence of the meningioma. Epidemiological studies have shown that a history of head trauma, smoking, and cell phone use has not been shown to be associated with an increased risk of the meningioma.

Currently, the standard protocol for treating meningioma generally includes surgery and radiotherapy. Although progress has been made in surgery and radiotherapy, most patients can be cured, but the recurrence rate of the malignant meningioma is as high as 50% to 80%, and the median survival time of some patients is less than 2 years. The disease is very difficult to treat. So far, chemotherapy, hormone therapy and immunotherapy have had very limited efficacy in patients with the malignant meningiomas; due to the particularity of brain tumors, surgical treatment for recurrent patients is also very limited; and surgery, radiotherapy and chemotherapy make patients poorly tolerated. With the aging of the population, the incidence of the meningioma increases. Effective therapies for meningiomas with good safety and tolerability are becoming more clinically important and urgently needed to be developed.

### Summary

Regarding the deficiencies in the treatment of meningiomas in the prior art, the purpose of the present invention is to develop a method that can effectively treat meningiomas, and the method has good safety and tolerance. Any reference herein to methods of treatment of a human body by therapy are to be interpreted as references to the compound or the composition herein described for use in such methods. Methods of medical treatment do not form part of the invention.

Honokiol, the English name is Honokiol, the chemical name is 3',5-di-2-propenyl-1,1'-biphenyl-2,4'-diphenol, and the structural formula is as shown in formula (I):

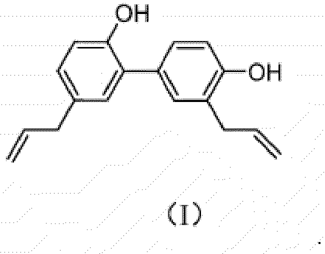

Honokiol is a small molecular compound with a wide range of biological activities extracted and isolated from the bark of Magnolia officinalis Rehd.etWils. The main biological activities of honokiol include anti-inflammation, anti-microbial, anti-ulcer, anti-oxidation, anti-anxiety, anti-depression, anti-thrombotic, anti-aging and cholesterol-lowering, etc.

Regarding the extensive medicinal value of honokiol, the inventor further studied the new use of honokiol. In clinical research, the inventor found that honokiol can effectively inhibit the growth of the meningioma, and eliminate and/or reduce lesions of the meningioma.

Therefore, one of the purposes of the present invention is to provide honokiol for use in treating meningioma.

According to the use for treating the meningioma of the present invention, the honokiol is prepared as a honokiol liposome, preferably a honokiol liposome for injection.

According to the use for treating the meningioma of the present invention, the honokiol inhibits the cell proliferation of the meningioma.

Another object of the present invention is to provide a honokiol liposome for use in treating meningioma.

According to the honokiol liposome of the present invention, the honokiol liposome is the honokiol liposome for injection.

According to the honokiol liposome of the present invention, the honokiol liposome includes following dosage forms: freeze-dried powder preparations, including injection freeze-dried powder preparations and oral freeze-dried powder preparations; tablets, including immediate-release tablets and sustained-release tablets; capsules, including hard capsules, soft capsules, sustained-release capsules and enteric-coated capsules; or transdermal preparations, etc.

According to the honokiol liposome of the present invention, the honokiol liposome can be administered through following routes: intravenous injection, intramuscular injection, subcutaneous injection, oral administration, eye administration, pulmonary administration, transdermal administration and nasal administration, etc.

### Beneficial effect

It has been found in clinical trials that honokiol can effectively treat the meningioma, effectively inhibit the growth of the meningioma, and eliminate and/or reduce lesions of the meningioma. Moreover, clinical trials have shown that honokiol has good safety and tolerance.

### Description of drawings

Figures 1A to 1C are the cranial magnetic resonance images of the meningioma patient before treatment with honokiol and after one cycle and three cycles of treatment with honokiol in Example 1, respectively.
Figures 2A to 2C are the cranial magnetic resonance images of the meningioma patient before treatment with honokiol and after one cycle and three cycles of treatment with honokiol in Example 2, respectively.
Figures 3A to 3C are the cranial magnetic resonance images of the meningioma patient before treatment with honokiol and after one cycle and three cycles of treatment with honokiol in Example 3, respectively.
Figures 4A to 4C are the cranial magnetic resonance images of the meningioma patient before treatment with honokiol and after one cycle and three cycles of treatment with honokiol in Example 4, respectively.

### Detailed description

Examples are provided below to further illustrate the use of honokiol in the preparation of a drug for treating meningioma.

The abbreviations of the technical terms involved in the examples are listed in Table 1 below.

**Table 1**

| | |
|---|---|
| ALT | alanine aminotransferase |
| APTT | activated partial thromboplastin time |
| AST | aspartate aminotransferase |
| CCr | creatinine clearance rate |
| CD34 | cluster of differentiation 34 |
| CD99 | cluster of differentiation 99 |
| CgA | chromogranin A |
| CK | cytokeratin |
| CPA | cerebellopontine angle |
| CTCAE | Common Terminology Criteria for Adverse Events |
| DLT | dose-limiting toxicity |
| ECOG | Eastern Cooperative Oncology Group |
| EMA | epithelial membrane antigen |
| Ki67 | cell proliferation marker |
| KPS | Karnofsky physical score |
| LVEF | left ventricular ejection fraction |
| MR | magnetic resonance |
| PR | progesterone receptor |
| PT | prothrombin time |
| RANO | response assessment in neuro-oncology |
| RECIST1.1 | Response Evaluation Criteria in Solid Tumors Version 1.1 |
| S100 | nervous tissue-origin marker |
| SAE | serious adverse event |
| SD | stable disease |
| SSTR | somatostatin receptor |
| Syn | synaptophysin |
| TT | thrombin time |
| ULN | upper limit of normal |

The test material used in the example is as follows:
Honokiol liposome for injection is derived from Chengdu JinRui Foundation Biotechnology Co., Ltd., or can be prepared according to the following methods: 50mg of honokiol, 500mg of soybean lecithin, 200mg of cholesterol, and 200mg of peony phosphatidylethanolamine were dissolved in 50mL of absolute ethanol for complete dissolution, the obtained solution was injected into 300mL of purified water and stirred, ethanol was then removed by rotary evaporation, and 800mg of sucrose as a freeze-dried excipient was added, lyophilized.

### Example 1

Description of the subject:
male, 20 years old;
on January 20, 2018, the subject's cranial MR showed the space-occupying of CPA on the right side and the jugular foramen region;
on November 27, 2018, the subject underwent the resection of a deep skull bottom lesion, endothelial meningoma, and local lesion being hemangioma-type meningoma, accompanied by bleeding, showing the non-typical meningioma, infiltrating bone tissue, comprehensive to malignant meningoma; immunohistochemistry: EMA (positive), PR (scattered in a little positive), SSTR (focal positive), CD34 (blood vessel positive), CD99 (+), S100 (-), Syn (-), CgA (-) , CK (occasionally positive), Ki67 (about 8-20 %);
in February 2019, the subject was treated for intracranial infection;
in June 2019, the subject received whole brain radiotherapy;
on March 5, 2021, the subject re-examined the cranial MR and the tumor recurred; in order to further treat, at the outpatient of Beijing Tiantan Hospital, Capital Medical University, the subject was included in the comprehensive therapy ward of neural tumor for "malignant tumor maintenance treatment";
on May 13, 2021, the subject entered the group "Phase I clinical trial of the clinical safety and tolerance of the injecting honokiol liposome (HK) for the treatments of the patients with advanced malignant solid tumors", the selected standard is as follows:
   1) $18 years old ⩽ age ⩽ 80 years old ,$ gender is not limited;
   2) for the patients with advanced malignant solid tumors diagnosed by histology or cytology, the previous standard treatment plans failed, they cannot tolerate or refuse existing therapy, preferably tumor species such as lung cancer, liver cancer, glioma, ovarian cancer, and colon cancer;
   3) according to the RECIST 1.1 standard (or RANO standard), there are evaluated tumor lesions;
   4) except for glioma, patients with brain metastases selected for other tumor species must meet the following conditions: no clinical symptoms related to brain metastases, no need for systemic corticosteroids or anticonvulsant drug treatment; patients with brain metastases who have been treated must be no progress by re-examination after 28 days at the end of treatment; no risk of intracerebral hemorrhage;
   5) those without severe hematopoietic dysfunction (absolute value of neutrophils ≥ 1.5x10⁹/L, platelets ≥ 80x10⁹ /L, hemoglobin ≥ 100g/L);
   6) those without serious organic disease of heart, lung, liver and kidney (LVEF (left ventricular ejection fraction) ≥ 50%; total bilirubin≤1.5×ULN; alanine aminotransferase (ALT) ≤ 2.5 × ULN ( ≤ 5 × ULN if there is liver metastasis); aspartate aminotransferase (AST)≤2.5×ULN (≤5×ULN if there is liver metastasis); serum creatinine≤1.5×ULN or CCr>40mL/min);
   7) those without severe coagulation abnormalities (PT≤1.5×ULN, APTT ≤1.5×ULN, TT≤1.5×ULN);
   8) at least 4 weeks have passed since the patients received the last anti-tumor treatment (chemotherapy, radiotherapy, biological therapy, hormone therapy and targeted therapy) before entering the group;
   9) $expected survival time ⩾ 12 weeks ;$
   10) for patients with glioma, KPS score > 50 points;
   11) for patients with other solid tumors, the ECOG score≤1;
   12) those who agree to participate in this study and sign the informed consent form.

Three cycles of medication were given to the subject: a cycle of 28 days, in which 5 days of continuous administration per week, 2 days of drug withdrawal, a period of 3 weeks, and 1 week of rest; honokiol liposome for injection was administered, and the dosage is 420mg/day honokiol; the method of administration is administration via peripherally inserted central venous catheters, and the infusion time is 2 hours.

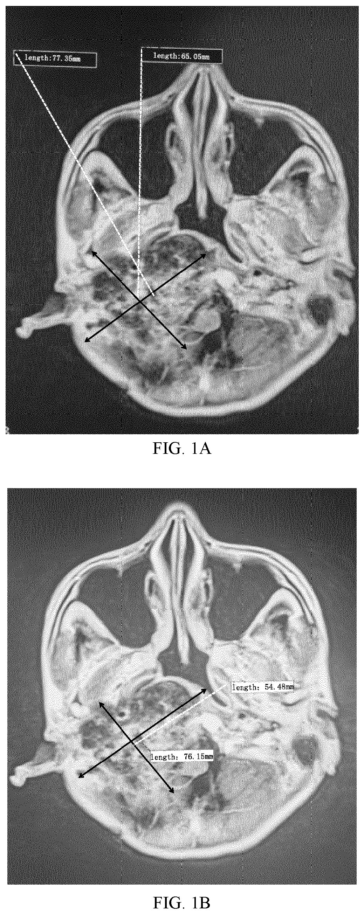

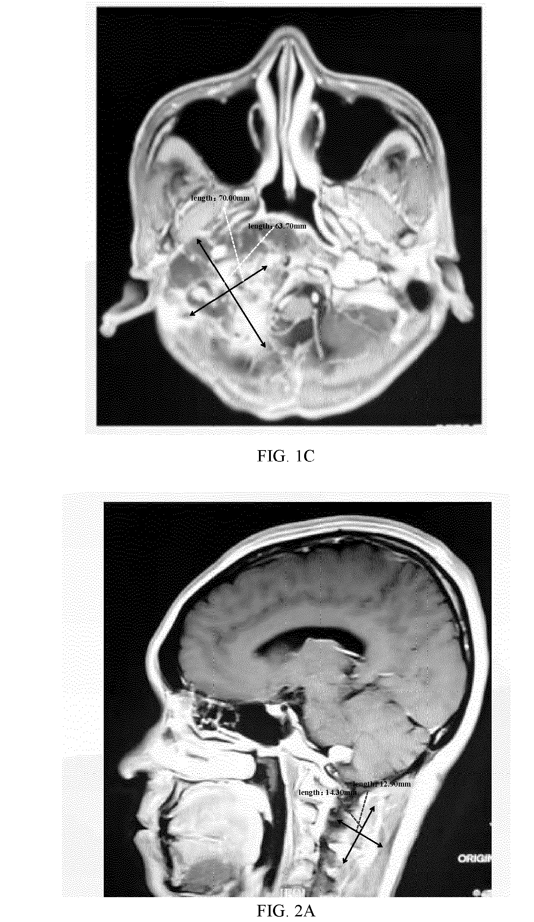

After completing the one cycle of medication, cranial MR re-examination was conducted. Figure 1A and Figure 1B show the cranial MR comparison images before and after the treatment. According to the RANO standard, the target lesion was reduced by 17.46% compared with that before treatment, and the product of the two perpendicular diameters of the target lesion was reduced from 77.3mm×65mm before treatment to 76.1mmX54.5mm. After completing the three cycles of medication, the cranial MR re-examination was conducted, and the cranial MR image is shown in Figure 1C. According to the RANO standard, it showed that the target lesion was reduced by 11.13% compared with that before treatment, and the product of the two perpendicular diameters of the target lesion was reduced to 70.1mm×63.7mm. The curative effect was evaluated as stable disease (SD), with improvement in clinical performance and no adverse reaction.

As of May 31, 2021, according to the inclusion standard of the above "Phase I clinical trial of the clinical safety and tolerance of the injecting honokiol liposome (HK) for the treatments of the patients with advanced malignant solid tumors" in Example 1, 37 subjects were included in the safety analysis set, and were administered different doses of honokiol liposomes for injection. The doses were 20 mg/day for 4 cases, 40 mg/day for 3 cases, 80 mg/day for 4 cases, 140 mg/day for 3 cases, 210 mg/day for 5 cases, 300 mg/day for 6 cases, and 420 mg/day for 12 cases. The administration method is administration via peripherally inserted central venous catheters, and the infusion time is 2 hours. Every 4 weeks is one administration cycle, 3 weeks per administration cycle (the first week to the third week of this cycle), 5 days of continuous administration per week, once a day, 2 days of drug withdrawal, and 1 week of rest.

After administration of honokiol liposome for injection, the main adverse reactions of the subjects were neutropenia, constipation, anemia, increased ALT, decreased fibrinogen, headache, dizziness, and hypokalemia. Most of these adverse reactions were mild. There was 1 case of DLT (CTCAE grade 3 increased ALT), and 4 cases of SAE were observed, all of which were remission, and there were no suspicious unexpected serious adverse reactions. Adverse events, adverse reactions, incidence of SAE, subjects' vital signs, physical examination, laboratory examination, 12-lead electrocardiogram, echocardiogram and other clinically significant changes were not evidenced by an increasing trend with increasing dose.

Clinical trials have shown that honokiol liposomes for injection can not only effectively treat the meningioma, but also have good safety and tolerance.

### Example 2

Description of the subject:
female, 39 years old;
One year before admission, the patient had pain and discomfort in the neck and pillow area without obvious incentives, which aggravated after exercise, without limb movement disorders, and without obvious upper limb radiating pain, which aggravated at night. For further treatment, she went to the Second Hospital of Dalian Medical University, and a cranial MRI showed a space occupying of the medulla. During the perioperative treatment, she went to the Second Hospital of Dalian Medical University. On January 10, 2020, lower brainstem tumor resection under general anesthesia was performed in this hospital. During the operation, the tumor size was about 1.5*1.5cm, accompanied by tumor hemorrhage, rotten fish-like, rich blood supply, and the tumor was completely resected under the microscope. Postoperative pathology: non-typical meningioma (WHO-II), KI-67 (25%). Hoarseness occurred postoperatively.

Before admission, she felt dizzy, weak, and occasionally coughed. She went to Tiantan Hospital, and a cranial MRI was performed 1 week before admission, showing that the medullary lesions had progressed. Two cycles of self-provided PD-1 immunotherapy were given, and the process went smoothly. Now for further treatment, the outpatient is hospitalized with "meningioma".

On October 14, 2021, as described in Example 1, "Phase I clinical trial of the clinical safety and tolerance of the injecting honokiol liposome (HK) for the treatments of the patients with advanced malignant solid tumors", she was selected into the standard group for administration.

Three cycles of medication were given to the subject: a cycle of 28 days, in which 5 days of continuous administration per week, 2 days of drug withdrawal, a period of 3 weeks, and 1 week of rest; honokiol liposome for injection was administered, and the dosage is 420mg/day honokiol; the method of administration is administration via peripherally inserted central venous catheters, and the infusion time is 2 hours.

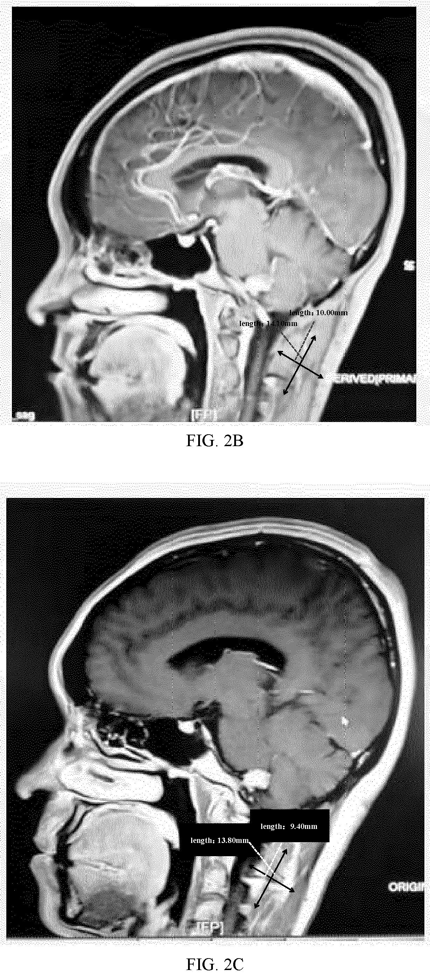

After completing the one cycle of medication, the cranial MR re-examination was conducted. Figure 2A and Figure 2B show the cranial MR comparison images before and after the treatment. According to the RANO standard, the target lesion was reduced by 22.97% compared with that before treatment, and the product of the two perpendicular diameters of the target lesion was reduced from 14.3mm×12.9mm before treatment to 14.1mm×10mm. After completing the three cycles of medication, the cranial MR re-examination was conducted, and the cranial MR image is shown in Figure 2C. According to the RANO standard, it showed that the target lesion was reduced by 29.13% compared with that before treatment, and the product of the two perpendicular diameters of the target lesion was reduced to 13.8mm×9.4mm. The curative effect was evaluated as stable disease (SD), with clinical manifestations of stability and tolerance to the drug.

### Example 3

Description of the subject:
male, 53 years old;
Five years before admission, the patient had no obvious cause of "twitching of the limbs accompanied by foaming at the mouth, and rolling his eyes for 4 hours", no memory loss, no headache, dizziness, no nausea, vomiting, no limb movement disorder, no hearing and vision loss. The cranial MRI showed intracranial space occupying, and he went to the First People's Hospital of Jining to complete relevant examinations. On February 21, 2016, a right frontal tumor resection was performed, and the postoperative pathology: meningioma. Gamma knife treatment was performed once in Shandong Armed Police General Hospital after operation. Cranioplasty was performed in October 2017.

On February 13, 2019, an epileptic seizure occurred, and the tumor recurrence was considered by re-examination. On February 18, 2019, the embolization of pelvic artery of the meningioma was performed. On February 21, 2019, the original incision approach tumor resection was performed. Postoperative pathology: meningioma (WHO I), postoperative whole brain radiotherapy was given. In April 2021, the tumor recurrence was considered by re-examination and no treatment was given. In May 2021, an epileptic seizure occurred and Debakin was given for treatment. In June 2021, he had a major epileptic seizure, which improved after symptomatic treatment, and the cranial MRI showed larger tumor than before.

On July 21, 2021, resection of the tumor next to the right frontal lobe was performed again. Postoperative pathology: anaplastic meningioma (WHO III), Ki-67 (50%). On August 17, 2021, one cycle of tislelizumab was administered. For further treatment, the outpatient is hospitalized with "meningioma".

On October 21, 2021, as described in Example 1, "Phase I clinical trial of the clinical safety and tolerance of the injecting honokiol liposome (HK) for the treatments of the patients with advanced malignant solid tumors", he was selected into the standard group for administration.

Three cycles of medication were given to the subject: a cycle of 28 days, in which 5 days of continuous administration per week, 2 days of drug withdrawal, a period of 3 weeks, and 1 week of rest; honokiol liposome for injection was administered, and the dosage is 420mg/day honokiol; the method of administration is administration via peripherally inserted central venous catheters, and the infusion time is 2 hours.

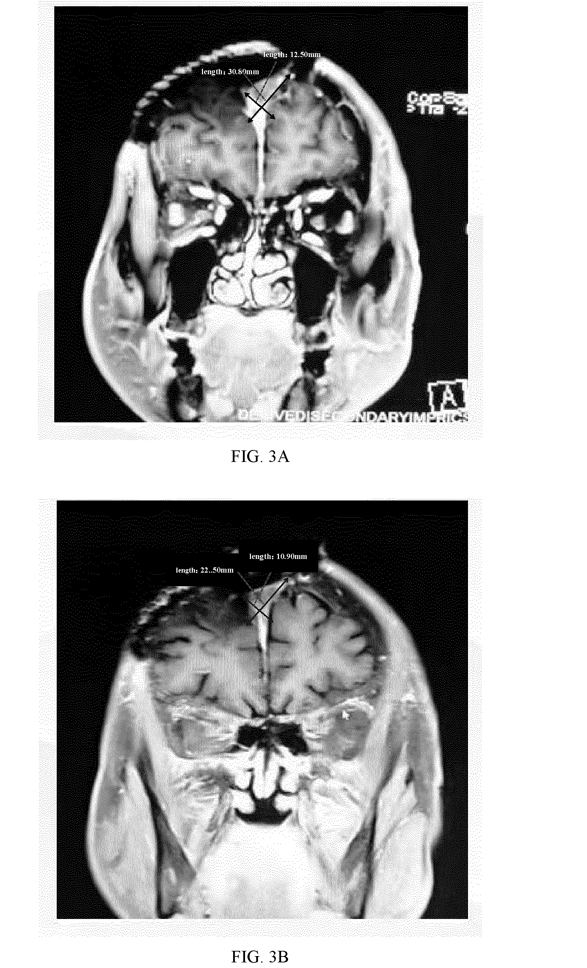

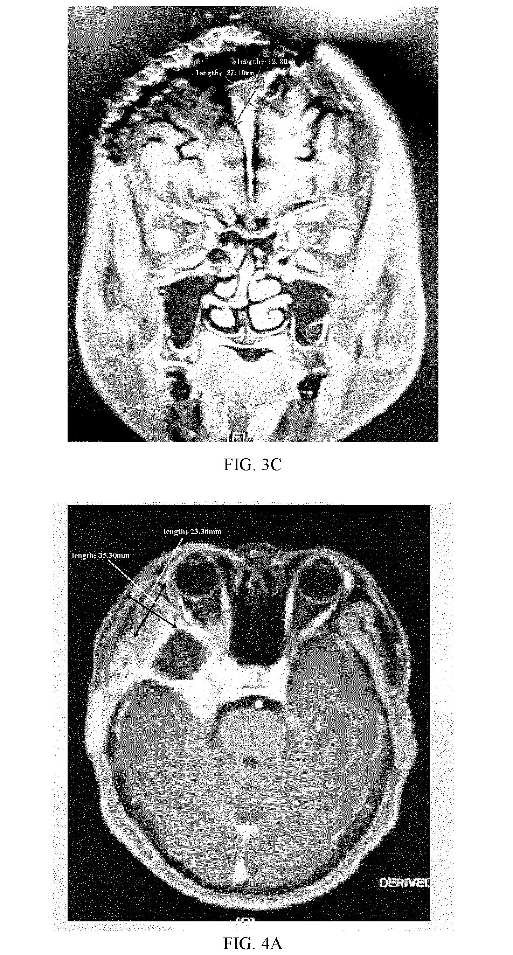

After completing the one cycle of medication, the cranial MR re-examination was conducted. Figure 3A and Figure 3B show the cranial MR comparison images before and after the treatment. According to the RANO standard, the target lesion was reduced by 35.30% compared with that before treatment, and the product of the two perpendicular diameters of the target lesion was reduced from 12.5mm × 30.8mm before treatment to 10.9mm× 22.5mm. After completing the three cycles of medication, the cranial MR re-examination was conducted, and the cranial MR image is shown in Figure 3C. According to the RANO standard, it showed that the target lesion was reduced by 13.42% compared with that before treatment, and the product of the two perpendicular diameters of the target lesion was reduced to 12.3mm × 27.1mm. The curative effect was evaluated as stable disease (SD), with improvement in clinical manifestations and tolerance to the drug.

### Example 4

Description of the subject:
female, 53 years old;
The patient went to the local hospital in 2012 due to epileptic seizures, and the examination revealed an intracranial space occupying. Right frontotemporal craniotomy and space occupying resection was performed, postoperative pathology: low meningioma of right middle cranial fossa (WHO I), regular re-examination every year after operation.

In 2016 and 2018, Gamma Knife treatments were performed due to recurrence. One year ago, the vision on the right side gradually declined, and then intermittent headaches, no limb convulsions, no incontinence, no nausea and vomiting, no limb movement disorders. She went to the local hospital 3 months before admission, the cranial MRI was performed, and it was found that the postoperative changes of the right frontotemporal craniotomy, space occupying in the right sphenoid ridge, middle cranial fossa, infratemporal fossa and right orbit: recurrence of the meningioma with multiple enhancements at the right orbital apex, anterior cranial fossa, and right tentorial marginal; multiple cerebral ischemic white matter lesions. Pathology: anaplastic meningioma (WHO III), Ki-67 (about 40-60%). For further treatment, the outpatient is hospitalized with "meningioma".

On October 28, 2021, as described in Example 1, "Phase I clinical trial of the clinical safety and tolerance of the injecting honokiol liposome (HK) for the treatments of the patients with advanced malignant solid tumors", she was selected into the standard group for administration.

Three cycles of medication were given to the subject: a cycle of 28 days, in which 5 days of continuous administration per week, 2 days of drug withdrawal, a period of 3 weeks, and 1 week of rest; honokiol liposome for injection was administered, and the dosage is 420mg/day honokiol; the method of administration is administration via peripherally inserted central venous catheters, and the infusion time is 2 hours.

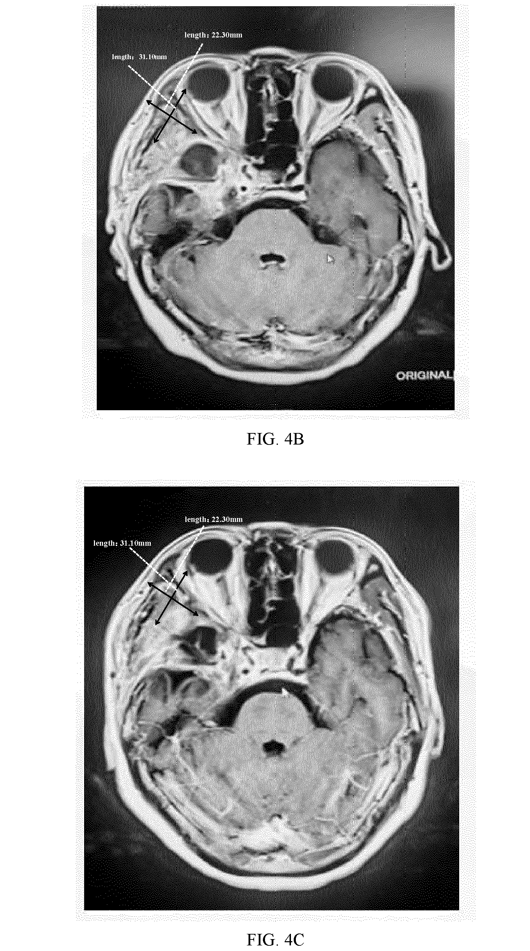

After completing the one cycle of medication, the cranial MR re-examination was conducted. Figure 4A and Figure 4B show the cranial MR comparison images before and after the treatment. According to the RANO standard, the target lesion was reduced by 15.68% compared with that before treatment, and the product of the two perpendicular diameters of the target lesion was reduced from 23.3mm × 35.3mm before treatment to 22.3mm × 31.1mm. After completing the three cycles of medication, the cranial MR re-examination was conducted, and the cranial MR image is shown in Figure 4C. According to the RANO standard, it showed that the target lesion was reduced by 15.68% compared with that before treatment, and the product of the two perpendicular diameters of the target lesion was reduced to 22.3mm × 31.1mm. The curative effect was evaluated as stable disease (SD), with improvement in clinical manifestations and tolerance to the drug.

## Claims

1. Honokiol for use in treating meningioma.

2. Honokiol for use according to claim 1, wherein the honokiol is prepared as a honokiol liposome.

3. Honokiol for use according to claim 2, wherein the honokiol liposome is a honokiol liposome for injection.

4. Honokiol for use according to any one of claims 1-3, wherein the honokiol inhibits a cell proliferation of the meningioma.

5. A honokiol liposome for use in treating meningioma.

6. The honokiol liposome for use according to claim 5, wherein the honokiol liposome is a honokiol liposome for injection.

7. The honokiol liposome for use according to claim 5, wherein the honokiol liposome comprises the following dosage forms: freeze-dried powder preparations; tablets; capsules; or transdermal preparations.

8. The honokiol liposome for use according to claim 7, wherein the freeze-dried powder preparations are selected from injection freeze-dried powder preparations and oral freeze-dried powder preparations.

9. The honokiol liposome for use according to claim 7, wherein the tablets are selected from immediate release tablets and sustained release tablets.

10. The honokiol liposome for use according to claim 7, wherein the capsules are selected from hard capsules, soft capsules, sustained release capsules and enteric coated capsules.

11. The honokiol liposome for use according to claim 5, wherein the honokiol liposome is administered through following routes: intravenous injection, intramuscular injection, subcutaneous injection, oral administration, eye administration, pulmonary administration, transdermal administration and nasal administration.

## Patentansprüche

1. Honokiol zur Verwendung beim Behandeln von Meningeomen.

2. Honokiol zur Verwendung nach Anspruch 1, wobei das Honokiol als Honokiol-Liposom hergestellt ist.

3. Honokiol zur Verwendung nach Anspruch 2, wobei das Honokiol-Liposom ein Honokiol-Liposom zur Injektion ist.

4. Honokiol zur Verwendung nach einem der Ansprüche 1-3, wobei das Honokiol die Zellproliferation des Meningeoms hemmt.

5. Honokiol-Liposom zur Verwendung beim Behandeln von Meningeomen.

6. Honokiol-Liposom zur Verwendung nach Anspruch 5, wobei das Honokiol-Liposom ein Honokiol-Liposom zur Injektion ist.

7. Honokiol-Liposom zur Verwendung nach Anspruch 5, wobei das Honokiol-Liposom die folgenden Darreichungsformen umfasst: gefriergetrocknete Pulverzubereitungen; Tabletten; Kapseln; oder transdermale Zubereitungen.

8. Honokiol-Liposom zur Verwendung nach Anspruch 7, wobei die gefriergetrockneten Pulverzubereitungen aus gefriergetrockneten Pulverzubereitungen zur Injektion und oralen gefriergetrockneten Pulverzubereitungen ausgewählt sind.

9. Honokiol-Liposom zur Verwendung nach Anspruch 7, wobei die Tabletten aus Tabletten mit sofortiger Wirkstofffreisetzung und Retardtabletten ausgewählt sind.

10. Honokiol-Liposom zur Verwendung nach Anspruch 7, wobei die Kapseln aus Hartkapseln, Weichkapseln, Retardkapseln und magensaftresistenten Kapseln ausgewählt sind.

11. Honokiol-Liposom zur Verwendung nach Anspruch 5, wobei das Honokiol-Liposom über folgende Verabreichungswege verabreicht wird: intravenöse Injektion, intramuskuläre Injektion, subkutane Injektion, orale Verabreichung, okuläre Verabreichung, pulmonale Verabreichung, transdermale Verabreichung und nasale Verabreichung.

## Revendications

1. Honokiol destiné à être utilisé dans le traitement d'un méningiome.

2. Honokiol destiné à être utilisé selon la revendication 1, dans lequel l'honokiol est préparé sous la forme d'un liposome d'honokiol.

3. Honokiol destiné à être utilisé selon la revendication 2, dans lequel le liposome d'honokiol est un liposome d'honokiol pour injection.

4. Honokiol destiné à être utilisé selon l'une quelconque des revendications 1 à 3, dans lequel l'honokiol inhibe une prolifération cellulaire du méningiome.

5. Liposome d'honokiol destiné à être utilisé dans le traitement d'un méningiome.

6. Liposome d'honokiol destiné à être utilisé selon la revendication 5, dans lequel le liposome d'honokiol est un liposome d'honokiol pour injection.

7. Liposome d'honokiol destiné à être utilisé selon la revendication 5, dans lequel le liposome d'honokiol comprend les formes posologiques suivantes : préparations en poudre lyophilisée ; comprimés ; capsules ; ou préparations transdermiques.

8. Liposome d'honokiol destiné à être utilisé selon la revendication 7, dans lequel les préparations en poudre lyophilisée sont choisies parmi des préparations en poudre lyophilisée pour injection et des préparations en poudre lyophilisée pour administration orale.

9. Liposome d'honokiol destiné à être utilisé selon la revendication 7, dans lequel les comprimés sont choisis parmi des comprimés à libération immédiate et des comprimés à libération prolongée.

10. Liposome d'honokiol destiné à être utilisé selon la revendication 7, dans lequel les capsules sont choisies parmi des capsules dures, des capsules molles, des capsules à libération prolongée et des capsules entérosolubles.

11. Liposome d'honokiol destiné à être utilisé selon la revendication 5, dans lequel le liposome d'honokiol est administré par les voies d'administration suivantes : injection intraveineuse, injection intramusculaire, injection sous-cutanée, administration orale, administration oculaire, administration pulmonaire, administration transdermique et administration nasale.
